# FASCICULE DE BREVET EUROPEEN

(11) **EP 1 573 336 B1**
(45) Date de publication et mention de la délivrance du brevet: **18.02.2009**
(21) Numéro de dépôt: 03799681.6
(22) Date de dépôt: 19.12.2003
(51) Int. Cl.: G01N 33/68

(54) **PROCEDE DE DETECTION DE LA PRP UTILISANT UN ANTIBIOTIQUE DES AMINOGLYCOSIDES DU GROUPE II**
VERFAHREN ZUR DETEKTION VON PRPSC UNTER VERWENDUNG VON GRUPPE II AMINOGLYKOSID ANTIBIOTIKA
PRP DETECTION METHOD USING A GROUP II AMINOGLYCOSIDE ANTIBIOTIC

(30) Priorité: 20.12.2002 FR 0216382
(43) Date de publication de la demande: 14.09.2005
(73) Titulaire: Agence Française de Securité Sanitaire des Aliments, 94701 Maisons-Alfort Cedex (FR); CENTRE NATIONAL DE LA RECHERCHE SCIENTIFIQUE (CNRS), 75794 Paris Cedex 16 (FR); Université Claude Bernard Lyon, 69622 Villeurbanne Cédex (FR); BIOMERIEUX, 69280 Marcy-L'Etoile (FR)
(72) Inventeur: MOUSSA, Aly, F-69600 Oullins (FR); COLEMAN, Anthony, William, F-69300 Calluire-Et-Cuire (FR); BENCSIK,Reynier, Anna, F-38110 Saint-Clair de la Tour (FR); SHAHGALDIAN, Patrick, F-69003 Lyon (FR); PERRON, Hervé, F-69290 Saint-Genies-Les-Ollières (FR); MARTIN, Ambroise, F-69390 Charly (FR)
(74) Mandataire: Bredema
(86) Numéro de dépôt international: PCT/FR2003/003856
(87) Numéro de publication internationale: WO 2004/059321

(56) Documents cités:
- WO-A-01/23425
- WO-A-02/33420
- WO-A-02/086511
- US-B1- 6 221 614
- TAGLIAVINI F. ET AL: "Tetracycline affects abnormal properties of synthetic PrP peptides and PrPsc in vitro" JOURNAL OF MOLECULAR BIOLOGY, vol. 300, no. 5, 28 juillet 2000 (2000-07-28), pages 1309-1322, XP002257687
- MERK: "The Merk Index, 12th Edition. Page 1507" 1996, MERCK * page 1507 *
- D. YALA, A.S.MERAD, D.MOHAMEDI, M.N. OUAR KORICH: "Classification et mode d'action des antibiotiques" MÉDICINE DU MAGHREB, vol. 91, 2001, pages 5-12,

## Description

La présente invention concerne une méthode de précipitation totale de PrP^{sc} par le sulfate de streptomycine et son utilisation pour l'immunodétection ou l'élimination de la PrP^{sc} de liquides ou de solutions.

La protéine prion native ou normale, désignée PrP ou PrP^{c} pour la protéine prion cellulaire, est une glycoprotéine largement exprimée dans les cellules lymphoïdes et neuronales des mammifères.

Des changements conformationnels de PrP^{c} conduisent à l'apparition et à la propagation de la protéine pathogène PrP^{sc} qui est résistante à la protéinase K. Cette protéine pathogène peut être appelée PrP^{sc} ou PrP^{res} indifféremment. L'accumulation de PrP^{sc} dans les organes des animaux est à l'origine de nombreuses maladies et notamment de la tremblante des petits ruminants, de la maladie cachectisante chronique (ou chronic wasting disease 'CWD') de l'élan et de l'antilope, de l'encéphalopathie spongiforme bovine (ESB) et de la maladie de Creutzfeldt-Jacob (MCJ) chez l'homme.

L'apparition tardive après une période d'incubation de 2 à 6 ans et le lent développement des symptômes chez le bétail infecté par l'ESB a considérablement ralenti le développement de modèles épidémiologiques. L'ESB est transmissible par ingestion à l'homme et a conduit à l'apparition d'une nouvelle forme de la maladie de Creutzfeldt-Jacob (vMCJ).

La détection de la protéine pathogène PrP^{sc} est difficile chez les animaux infectés sains avant le développement de la maladie et surtout dans le sang et l'urine chez les animaux malades. Il est à présent établi que la PrP^{sc} présente chez les animaux destinés à l'alimentation humaine se transmet à l'homme lors de l'ingestion des tissus infectés. Un objectif majeur de santé publique est donc d'éviter cette transmission en détectant le PrP^{sc} chez des animaux destinés à la consommation humaine en vue de les retirer de la chaîne alimentaire.

La détection de la présence de PrP^{sc} dans des échantillons biologiques ou chez des animaux devient donc d'une extrême importance et plusieurs équipes de chercheurs développent des méthodes de détections immunologiques (WO02/086511). Par ailleurs, des méthodes pour complexer à la PrP^{sc} des peptides, des petites molécules ou des inhibiteurs en vue du traitement de vMCJ font l'objet de recherches actives. Toutefois, les méthodes en l'état de la technique se heurtent sans arrêt à la difficulté d'identifier la PrP^{sc} de manière fiable lorsqu'elle est en faible quantité dans un échantillon biologique.

Les inventeurs ont mis en évidence une nouvelle propriété des aminoglycosides, et notamment des aminoglycosides du groupe II et plus particulièrement de la streptomycine, et ont démontré la capacité de ces antibiotiques à se complexer avec la PrP^{sc} et à la faire précipiter.

Les inventeurs ont ainsi observé que l'ajout d'un aminoglycoside du groupe II, et plus particulièrement de la strptomycine, dans un échantillon biologique provenant de diverses origines animales et contenant des prions a pour conséquence de faire augmenter la masse moléculaire apparente des 3 bandes des prions. Cette observation et les expériences qui ont suivi leur ont permis de démontrer que l'aminoglycoside du groupe II se complexe avec la PrP^{sc} et que cette complexation a pour conséquence de précipiter la PrPsc et d'augmenter considérablement les possibilités de détection de la PrP^{sc} par rapport aux méthodes classiquement employées par l'homme du métier.

La présente invention a donc pour objet un procédé de concentration par précipitation de la PrPsc à utiliser lors du diagnostic des maladies dues aux prions ou pour l'élimination des prions présents dans un liquide biologique et caractérisé en ce que l'on met en présence une suspension du tissu ou un fluide biologique issu ou obtenu d'un organisme animal ou humain avec un antibiotique de préférence choisi dans la famille des aminoglycosides du groupe II et de préférence la streptomycine.

Plus précisément, le procédé selon l'invention comprend les étapes suivantes :
a) on ajoute, à la suspension d'un tissu ou d'un fluide biologique issu ou obtenu d'un organisme animal ou humain, un aminoglycoside du groupe II,
b) on place la solution dans un tampon adéquat, et on la soumet à un chauffage, puis on centrifuge la solution obtenue et l'on sépare le culot du surnageant,
c) on détecte la PrP^{sc} après migration sur un gel d'électrophorèse, transfert et immunodétection.

Le procédé de l'invention peut également comprendre une étape supplémentaire de digestion de l'échantillon à doser par une protéinase et notamment la protéinase K. Ainsi, le procédé de l'invention comprend les étapes suivantes :
a) on ajoute, à la suspension d'un tissu ou d'un fluide biologique issu ou obtenu d'un organisme animal ou humain, de la protéinase, par exemple de la protéinase K,
b) on met un aminoglycoside du groupe II,
c) on place la suspension dans un tampon adéquat, et on la soumet à un chauffage, puis on centrifuge la solution obtenue et l'on sépare le culot du surnageant,
d) on détecte la PrP^{sc} après migration sur un gel d'électrophorèse, transfert et immunodétection.

Plus précisément, le procédé de l'invention comprend les étapes suivantes :
a) préparation d'une suspension à 10 % par homogénéisation de fluide biologique ou de tissu obtenu d'organisme animal ou humain en 5% de solution de glucose,
b) on ajoute, aux 100 µl de la suspension obtenue, la protéinase K puis on incube à 37C° pendant une heure,
c) on ajoute, à la suspension, un aminoglycoside du groupe II puis on incube une deuxième heure à 37C°,
d) on place la suspension dans un tampon adéquat, et on la soumet à un chauffage, puis on centrifuge la solution obtenue et l'on sépare le culot du surnageant,
e) on suspend le culot dans 50 µl d'une solution de 50 % v/v d'urée 8M et du tampon Laemmli dénaturant comme décrit par Laemmli, Nature 227 (1970), 680-685. Après une agitation vortex vigoureuse, on chauffe 5 mn à 100°C, on centrifuge à 12000 g et on récupère les surnageants pour les faire migrer sur SDS PAGE,
f) après migration sur un gel d'électrophorèse, notamment une électrophorèse unidimensionnelle sur gel de polyacrylamide dodecyl sulfate 15% (SDS PAGE) comme décrit par Laemmli, Nature 227 (1970), 680-685, les protéines sont transférées par électrophorèse sur des membranes nitrocelluloses et immunoblottées à température ambiante pendant 60 minutes avec un anticorps monoclonal reconnaissant un épitope spécifique constitué des acides aminés 126-160. L'anticorps secondaire (1/5000) est un anticorps de chèvre dirigé contre les chaînes lourdes et légères des immunoglobulines de souris conjuguées à la peroxydase de raifort (IgG H+L) ; les blots sont ensuite lavés et les signaux sont détectés par chimioluminescence soit avec un kit ECL (Amersham) sur des films (Biomex light, Kodak) ou avec un super Signal Ultra (Pierce) et visualisation sur Fluor S. Multimager (BioRad)

Le procédé selon l'invention présente l'avantage de ne pas nécessiter d'ultracentrifugation. Le procédé selon l'invention concerne également la complexation de l'aminoglycoside du groupe II avec les protéines du prion et la précipitation des protéines du prion par ledit aminoglycoside.

Suivant un mode de réalisation de l'invention, le tissu biologique est issu ou obtenu à partir d'un cerveau ou d'un autre tissu animal ou humain, ou d'un fluide biologique tel que liquide céphalorachidien ou sérum.

La protéinase utilisée dans le procédé selon l'invention est une protéinase choisie pour sa capacité à digérer les protéines présentes incluant les formes normales de la protéine du prion, et pour son incapacité à digérer les formes pathologiques de cette protéine. Avantageusement, il s'agit de la protéinase K.

Suivant un mode de réalisation préféré de l'invention, le tissu préalablement à sa mise en contact avec ledit aminoglycoside du groupe II, est homogénéisé dans une solution de glucose. De préférence, il s'agit d'une solution de glucose à 5 %.

L'étape de chauffage après avoir ajouté 100 µl du tampon Laemmli à la suspension résultant de la mise en contact de l'échantillon biologique avec la protéinase K et l'aminoglycoside du groupe II, correspond à une augmentation de température comprise entre 60 et 150°C, de préférence d'environ 100°C.

Suivant un mode de réalisation préféré de l'invention, l'aminoglycoside du groupe II est la streptomycine ou un de ses dérivés.

L'invention a également pour objet l'utilisation d'un tel aminoglycoside du groupe II pour la précipitation, la détection même en immunohistochimie détection et/ou le diagnostic de la PrP^{sc}.

L'invention a encore pour objet l'utilisation d'un tel aminoglycoside du groupe II pour l'élimination de la PrP^{sc} d'un fluide biologique.

Enfin, l'invention se rapporte également à l'utilisation d'un kit de diagnostic des pathologies liées à la présence de PrP^{sc}, caractérisé en ce qu'il comprend un tel aminoglycoside du groupe II.

D'autres avantages et caractéristiques de l'invention apparaîtront à la lecture des exemples qui suivent concernant l'amplification de la détection du PrP^{sc} lorsque l'échantillon biologique est mis en présence d'un aminoglycoside du groupe II.

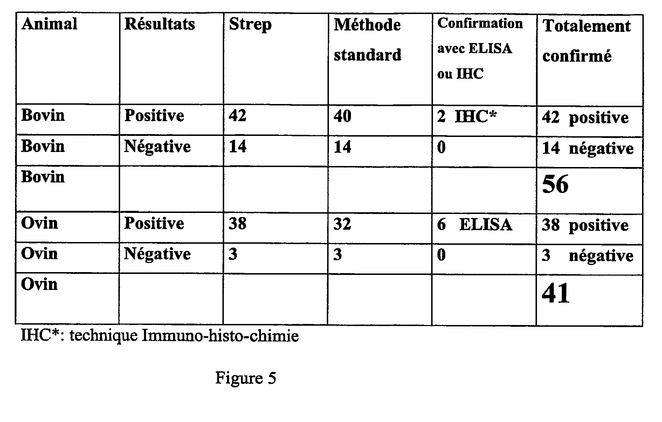

Il sera fait référence dans ces exemples aux dessins en annexe dans lesquels :
- la figure 1A est un exemple comparatif de la détection après électrophorèse sur gel de polyacrylamide à 15 %, transfert et immunodétection, de PrP^{sc} dans un échantillon de cerveau de mouton atteint de la tremblante et mis en présence de quantités de plus en plus importantes de streptomycine ;
- la figure 1B est un graphe de comparaison du poids moléculaire moyen mesuré de chaque bande PrP^{sc} de la figure 1B avant et après le mélange avec des quantités croissantes de streptomycine ;
- la figure 2 est un exemple comparatif de la détection après électrophorèse sur gel de polyacrylamide à 15 %, transfert et immunodétection, de PrP^{sc}, respectivement dans le surnageant et dans les précipités, obtenus à l'issue de la deuxième heure d'incubation d'une suspension très riche en protéine PrP^{sc} en absence et présence de quantité variable de streptomycine ;
- la figure 3 montre que l'addition en même temps de la protéinase K et de streptomycine à une suspension

contenant une faible quantité du PrP^{sc} a pour conséquence la disparition de la PrP^{sc} du surnageant et son apparition dans les précipités ;
- les figures 4A, 4B et 4C montrent, après électrophorèse sur gel de polyacrylamide à 15 %, transfert et immunodépressif, l'augmentation du seuil de détection de la PrP^{sc} lors de l'ajout de quantités croissantes de streptomycine ;
- le tableau de la figure 5 se réfère à l'exemple 5 et présente une comparaison des résultats du diagnostic du PrP^{sc} sur 97 cerveaux d'animaux obtenus par la technique selon l'invention avec ceux obtenus par la technique de référence ;
- la figure 6 montre des images en microscopie de sonde à balayage (SPM) en mode non-contact pour des films séchés de la protéine prion recombinante (recPrP) seule (A), de recPrP en présence de streptomycine (B) et de streptomycine seule (C) ;
- la figure 7A est un exemple comparatif de la détection après électrophorèse sur gel 12% bis-tris acrylmide, transfert et immunodétection, de la protéine prion dans des échantillons de liquide céphalo-rachidien (LCR) de patients atteints par la maladie de Creutzfeld-JaKob (MCJ+) et de patients non atteints par la MCJ (MJC-) traités par une gamme de streptomycine. Le tableau 7B résume les dépôts effectués dans les pistes de la figure 7A ;
- la figure 8A montre les résultats d'un western blot, d'échantillons de LCR(+) et de LCR (-) à la MJC digérés ou non par la protéinase K selon une gamme de concentration et traités ou non par la streptomycine. La révélation immunologique est assurée par un anticorps anti-prion. Le tableau 8B résume les dépôts effectués dans les pistes de la figure 8A.

Les échantillons biologiques sur lesquels les exemples 2 et 3 ci-dessous ont été réalisés ont été obtenus à partir d'homogénat de cerveau bovin placé dans une solution de glucose à 5 %. Des volumes de 100 µl de cette suspension, correspondant à 10 mg de tissu cérébral, sont utilisés pour les expériences qui suivent. Chaque échantillon est donc constitué de 100 µl, dans lesquels on ajoute de la protéinase K. Après une heure à 37°C on ajoute ou non de la streptomycine. La solution est agitée sous vortex et incubée à 37°C pendant une autre heure. Après avoir ajouté 100 µl de tampon Laemmli dénaturant, on chauffe à 100°C pendant 5 mn et on centrifuge à 12000 g pendant 5 minutes, puis on récupère les surnageants pour les faire migrer sur SDS-PAGE. Les culots sont également récupérés et on leur ajoute 50 µl d'un solution à 50% v/v d'urée 8M et le tampon de laemmli. Après une vigoureuse agitation sous vortex, on chauffe à 100°C pendant 5 mn et on centrifuge à 12000 g pendant 5 minutes, puis on récupère les surnageants pour les faire migrer sur SDS-PAGE.

### Exemple 1

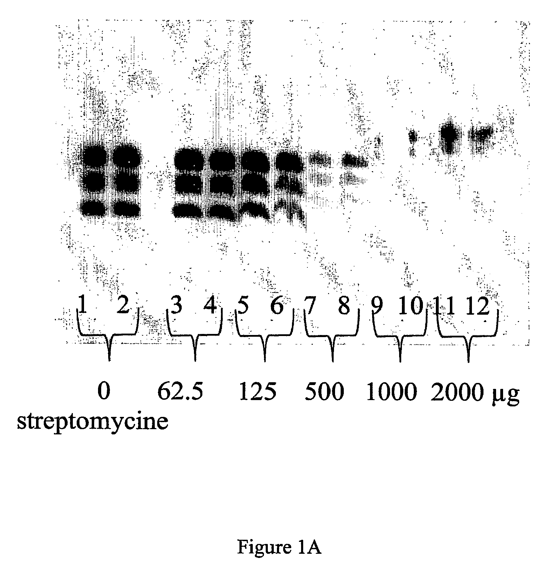

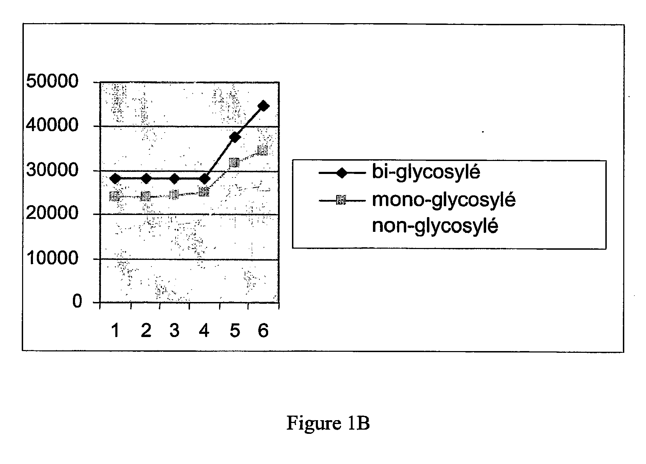

L'exemple 1 se réfère aux figures 1A et 1B relatives à des essais dans lesquels des concentrations croissantes (0 µg ; 62,5 µg ; 125 µg ; 500 µg et 2000 µg) de streptomycine sont ajoutées à des quantités constantes du PrP^{sc} extrait de l'équivalent de 920 µg de cerveau d'un mouton atteint de tremblante puis le mélange est centrifugé. Le surnageant est utilisé pour la détection immunologique par Western Blot (figure 1A) et la mesure de la masse moléculaire moyenne des bandes de PrP^{sc} (figure 1B). Les résultats montrent que l'accroissement de la quantité de streptomycine, à savoir un ajout de 0 ; 62,5, 125, 500 ,1000 et 2000 µg permet de constater qu'aux plus basses concentrations en streptomycine, la bande de la protéine non-glycosylée est la première à montrer une augmentation de la masse moléculaire apparente. Puis, la complexation concerne la bande de la protéine mono-glycosylée et finalement la protéine bi-glycosylée est complexée quand la concentration en streptomycine est la plus importante.

Le nombre de molécules de streptomycine liées à chacune des bandes de PrP^{sc} à une concentration donnée est évalué en mesurant le poids moléculaire de chaque bande. Les inventeurs ont estimé que l'augmentation du poids moléculaire apparente de chacune des bandes de PrP^{sc} en présence de 2000 µg de streptomycine correspond à l'accrochage de 10 à 12 molécules de streptomycine par bande de PrP^{sc}.

### Exemple 2

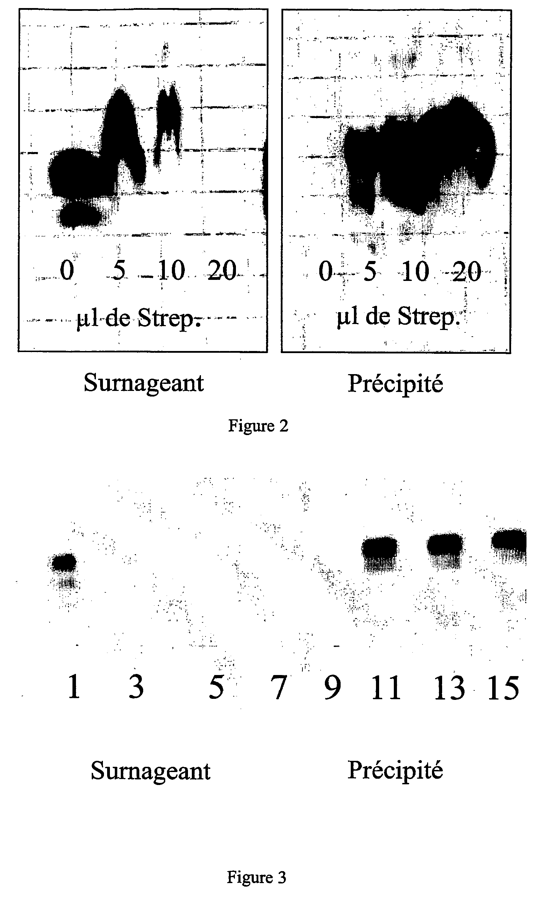

L'exemple 2 se réfère à la figure 2 relative à des essais dans lesquels des concentrations croissantes (0 µl ; 5 µl ; 10 µl ; 20 µl) de streptomycine à 1 g/ml sont ajoutées à des quantités constantes de l'échantillon biologique préparé comme indiqué ci-dessus. En absence de streptomycine, toutes les bandes PrP^{sc} sont identifiées comme étant dans le surnageant. Et progressivement, elles vont être détectées simultanément dans le culot et dans le surnageant. La quantité de PrP^{sc} présente dans le surnageant diminue progressivement pendant que la quantité augmente progressivement dans les précipités. L'addition de 20 µl de streptomycine précipite totalement le PrP^{sc} qui n'est alors détectée que dans les précipités.

### Exemple 3

L'exemple 3 se réfère à la figure 3.

On répète l'expérience de l'exemple 2 mais avec le même échantillon de cerveau dilué au 1/25 par rapport à l'exemple 2 et en diminuant la période d'incubation à une heure après l'addition simultanée de la protéinase K et la streptomycine aux suspensions de cerveau. Le résultat est que l'on ne détecte des bandes de PrP^{sc} que dans le surnageant en absence de streptomycine et que dans le culot en présence de n'importe quelle concentration de streptomycine.

### Exemple 4

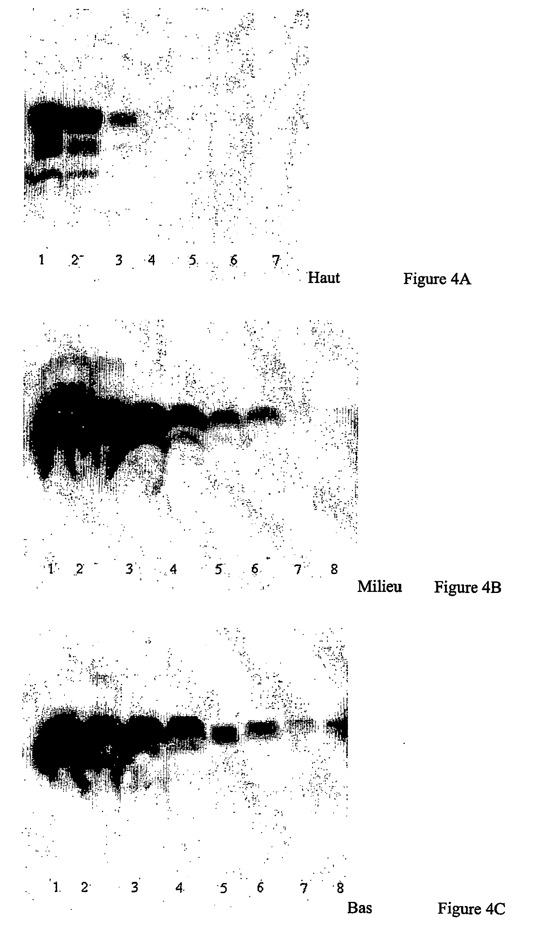

L'exemple 4 se réfère aux figures 4A, 4B et 4C.

A partir d'une dilution mère de 1:2 en série d'un homogénat à 10 % de cerveau bovin infecté par l'ESB dans une solution de glucose à 5 % on prépare 3 jeux de tubes contenant chacun 100 µl de la même dilution.

A chaque tube du premier jeu de dilution (de pure au 1/64)est ajouté 1 µg de protéinase K dans un volume de 10 µl.

A chaque tube du second jeu (de 1/2 au 1/256) sont ajoutés simultanément 5 µl de streptomycine et 1 µg de protéinase K dans un volume de 10µl.

A chaque tube du troisième jeu (de 1/2 au 1/256) sont ajoutés simultanément 10 µl de streptomycine et 1 µg de protéinase K dans un volume de 10 µl.

Tous les tubes sont incubés à 37°C pendant une heure puis on ajoute 100 µl de tampon Laemmli dénaturant. On chauffe à 100°C pendant cinq minutes on centrifuge à 12000 g pendant 5 minutes, et on récupère les surnageants du premier jeu de tubes pour déposition sur SDS-PAGE. Les surnageants des tubes du second et du troisième jeu de tubes sont éliminés et on ajoute dans chaque tube 50 µl d'urée 8M à 50%v/v et du tampon Laemmli dénaturant. Après une agitation vortex vigoureuse, on chauffe 5 mn à 100 °C on centrifuge à 12000 g et on récupère les surnageants pour les second et troisième jeux de tubes.

La figure 4A montre que la limite de détection de PrP^{sc} détectée en l'absence de streptomycine est au 1/16.

La figure 4B montre la quantité de PrP^{sc} précipitée en présence de 5 µl de streptomycine : la PrP^{sc} est détectable jusqu'à la dilution 1/128, ce qui montre un seuil de détection largement augmenté.

La figure 4C montre la quantité de PrP^{sc} précipitée en présence de 10 µl de streptomycine : la figure 4C montre que même sur les échantillons les plus dilués (1/256), la détection est possible et est représentative.

### Exemple 5 - Exemple comparatif

La technique de référence est basée sur l'extraction de la PrP^{sc} à partir de 1,2 ml d'homogénat cérébral (Madec et al., Microbial Pathogenesis, 28 (2000) 353-362). Les homogénats sont forcés à travers une aiguille de diamètre 0,4 mm avant d'être traités pendant une heure à 37°C par 10 µg de protéinase K par 100 mg de tissu cérébral. Après avoir ajouté du sarkosyl (10%) et 10 mM de tampon Tris (pH 7,4), les échantillons sont incubés pendant 15 mn à température ambiante puis centrifugés à 245,000 g pendant 4 heures à 20°C sur un coussin de saccharose à 10 % (Beckman TL 100 ultracentrifugeuse). Le culot est finalement remis en suspension dans 50 µl de tampon dénaturant Laemmli chauffé pendant 5 mn à 100°C et centrifugé encore pendant 5 mn à 12000 g. On récupère les surnageants pour migration sur SDS PAGE.

Selon la technique de l'invention, on utilise 100 µl de suspension cérébrale obtenue après broyage du cerveau décrit dans la technique de référence. On ajoute 1 µg de protéinase K et on incube une première fois pendant une heure. On ajoute ensuite 20 µl de streptomycine et on incube une seconde fois pendant une heure. On ajoute ensuite 100 µl de tampon dénaturant Laemmli. Après 5 mn de chauffage à 100°C, on centrifuge 2 mn à 12000 g. On élimine les surnageants, puis on ajoute 50 µl de 50 % v/v d'urée 8M et du tampon dénaturant Laemmli. Après une agitation vortex vigoureuse, on chauffe 5 mn à 100°C et on centrifuge encore pendant 2 mn à 12000 g. On récupère les surnageants pour la migration sur SDS PAGE.

Les résultats présents dans le tableau 1 : en conclusion, l'utilisation de sulfate de streptomycine permet une meilleure détection des cas faiblement positifs et de plus permet d'éviter une ultracentrifugation longue et coûteuse.

### Exemple 6

Des échantillons de recPrP (protéine prion recombinante) sont préparés en diluant une solution de recPrP (42 µM) avec un volume équivalent d'eau ou avec une solution de streptomycine (1 g/ml) ; la référence pour la streptomycine seule est préparée en utilisant une solution de 0,5 g/ml.

Les échantillons sont préparés par déposition de 10 µl de ces solutions sur du mica fraîchement clivé et séchage pendant 24 heures à 37°C.

On effectue une analyse par imagerie au moyen d'un Thermomicroscope Explorer AFM équipé avec un scanner trépied 100 µm, en mode non-contact, utilisant des fréquences de résonance élevées (F₀ = 320 kHz) de cantilever pyramidal avec des sondes silicones à une fréquence de balayage de 1 Hz. Le traitement des images est réalisé avec le logiciel SPMlab 5.1 et présenté non-filtrées.

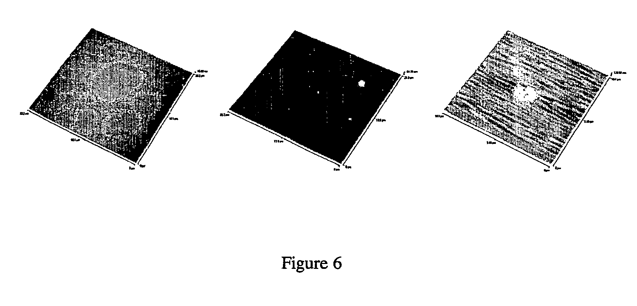

Il ressort de la figure 6 que les images de recPrP seuls montrent une structure en agrégats circulaires caractéristiques. Les films de streptomycine seule montrent une organisation de surface pseudo-cristalline. Les films contenant le mélange montrent une surface amorphe : aucune organisation cristalline ou globulaire n'est constatée. L'interaction entre la streptomycine et la PrP inhibe donc les propriétés d'organisation de surface des deux composants du mélange.

### Exemple 7

Des échantillons de prélèvements post-mortem de liquide céphalo-rachidien (LCR) de préférence non hémolysés et sans débris cellulaires sont prélevés chez des patients non atteints par la MJC et chez des patients atteints par la MJC. Un échantillon de LCR de patient non atteint par la MCJ et un échantillon de LCR de patient atteint par la MCJ sont mis au contact d'une solution de streptomycine, selon une gamme de concentration comprise entre 0,05g/mL et 0,2g/mL (0,05 - 0,1 et 0,2g/mL). Après homogénéisation au vortex, les échantillons sont incubés pendant 1 heure à 37°C puis sont ensuite centrifugés pendant 5 minutes à 12000g.

Les culots obtenus sont repris par du tampon d'extraction protéique. Après chauffage à 100°C pendant 10 minutes, les échantillons sont de nouveau centrifugés pendant 5 minutes à 12000 g. 15µL de chaque surnageant sont déposés sur gel SDS-PAGE 12 % bis-tris acrylamide. En parallèle, 5 µL d'un extrait de PrP^{sc} cérébrale dilué au 1/100 dans le tampon de dénaturation sont déposés en contrôle positif. Cet extrait est préparé selon le protocole de référence utilisé pour le diagnostic de la maladie de Creutzfeld-Jakob. La migration électrophorétique se réalise à voltage constant (200V) pendant 40 minutes dans du tampon de migration 1 fois concentré. Les protéines sont ensuite transférées sur une membrane de PVDF activée par système semi-sec entre deux électrodes de graphite, pendant 1 heure à puissance constante (1W). La révélation immunologique directe est ensuite assurée par l'anticorps anti-prion AC23 (qui reconnaît la région définie par les acides aminés 145-154 de la PrP humaine et les régions homologues des PrP animales) à 0,5µg/ml couplé à la peroxydase de raifort.

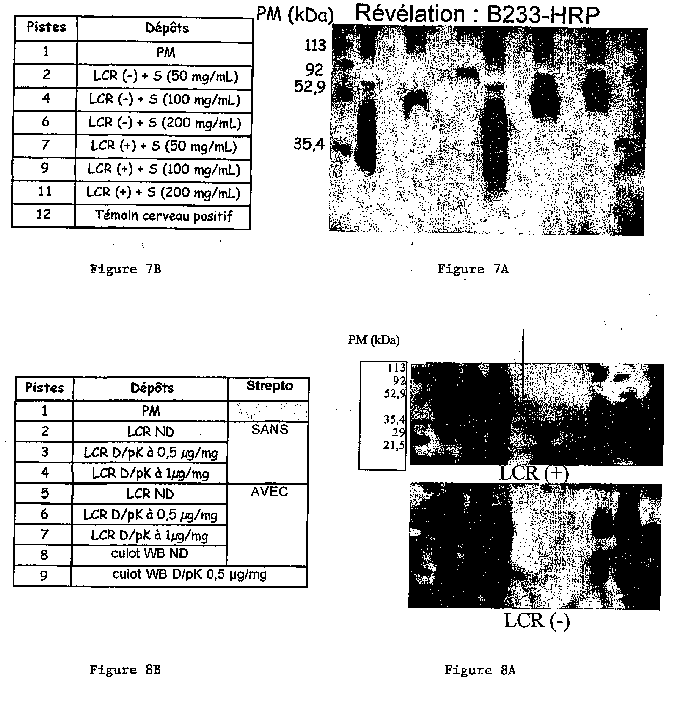

La figure 7A montre que la streptomycine se lie bien à la protéine prion en absence de digestion par la protéinase K. En effet, après traitement par la streptomycine à 0,1g/mL et 0,2g/mL, une seule bande est observée et son poids moléculaire apparent se décale d'une façon proportionnelle à la concentration de streptomycine. La taille apparente de cette bande est d'environ 50 kDa pour 0.1g/mL de streptomycine et approximativement de 80 kDa pour 0,2 g/mL de streptomycine. De plus, le profil de la bande (aspect incurvé, trainage) suggère une agrégation moléculaire.

### Exemple 8

Un LCR de patient non atteint par la MCJ et un LCR de patient atteint par la MCJ sont digérés par la protéinase K, utilisée à 0,5 µg/mL ou 1 µg/mL. En parallèle, un aliquot de chaque échantillon ne subit pas de digestion par la protéinase K. La digestion se réalise à 37°C pendant 1 heure sous agitation douce. Après la digestion, les échantillons sont incubés pendant 1 heure à 37°C en présence de streptomycine à 50 mg/mL de concentration finale, puis on procède à une centrifugation pendant 5 minutes à 12000g.

Les protéines sont ensuite extraites et dénaturées par chauffage en présence de tampon de dénaturation protéique puis elles sont analysées en western blot conformément au protocole de l'exemple 7. La révélation immunologique directe est réalisée à l'aide de l'anticorps AC23 (utilisé à 0,5 µg/ml) couplé à la peroxydase de raifort.

La figure 8A montre qu'une bande unique, de faible intensité, est observée lorsque l'échantillon est traité par la streptomycine. Cette bande, d'environ 35 kDa de masse moléculaire apparente, est visible pour l'échantillon négatif uniquement en absence de digestion par la protéinase K (piste 5 du gel LCR (-)) alors qu'elle est visible pour l'échantillon positif quelle que soit la concentration en protéinase K utilisée (pistes 5, 6 et 7 du gel LCR (+)), caractéristique de la forme résistante de la protéine prion.

La détection de la protéine prion dans le LCR est rendue possible par l'utilisation de la streptomycine. En effet, en absence de digestion par la protéinase K, la détection de la PrP totale (cellulaire et pathologique) est amplifiée en présence de streptomycine et, de manière inattendue, la PrP^{sc} est préférentiellement détectée. Après digestion par la protéinase K, cette technologie permet de mettre en évidence un signal significativement différent entre les LCR issus de patients non atteints par la maladie de Creutzfeldt-Jakob et les LCR issus de patients atteints par cette même maladie. La streptomycine présente donc un intérêt pour la détection de la PrP^{res} dans les fluides biologiques.

Par ailleurs, les aminoglycosides tels que la streptomycine peuvent être utilisés pour l'élimination de la PrP^{sc} du fait de la précipitation de la PrP^{sc} après contact avec les aminoglycosides.

## Revendications

1. Procédé de détection de la PrP, en particulier de la PrP^{sc}, **caractérisé en ce que** l'on met en présence un tissu ou un fluide biologique issu ou obtenu d'un organisme animal ou humain avec un antibiotique choisi dans la famille des aminoglycosides du groupe II.

2. Procédé selon la revendication 1, **caractérisé en ce que** :
a) on ajoute, à la suspension d'un tissu ou d'un fluide biologique issu ou obtenu d'un organisme animal ou humain, un aminoglycoside du groupe II,
b) on place la solution dans un tampon adéquat, et on la soumet à un chauffage, puis on centrifuge la solution obtenue et l'on sépare le culot du surnageant,
c) on détecte la PrP^{sc} après migration sur un gel d'électrophorèse, transfert et immunodétection.

3. Procédé selon la revendication 1, **caractérisé en ce que** :
a) on ajoute, à la suspension d'un tissu ou d'un fluide biologique issu ou obtenu d'un organisme animal ou humain, de la protéinase K,
b) on met un aminoglycoside du groupe II,
c) on place la suspension dans un tampon adéquat, et on la soumet à un chauffage, puis on centrifuge la solution obtenue et l'on sépare le culot du surnageant,
d) on détecte la PrP^{sc} après migration sur un gel d'électrophorèse, transfert et immunodétection.

4. Procédé selon l'une quelconque des revendications 1 à 3, **caractérisé en ce que** ledit tissu biologique est issu ou obtenu d'un cerveau ou autre tissu animal ou humain.

5. Procédé selon l'une quelconque des revendications 3 ou 4, **caractérisé en ce que** ladite protéinase est une protéinase K.

6. Procédé selon l'une quelconque des revendications 1 à 5, **caractérisé en ce que** le tissu ou le fluide biologique, préalablement à sa mise en contact avec ledit aminoglycoside, est homogénéisé dans une solution de glucose.

7. Procédé selon l'une quelconque des revendications 1 à 6, **caractérisé en ce que** l'étape de chauffage correspond à une augmentation de température comprise entre 60 et 150°C.

8. Procédé selon l'une quelconque des revendications 1 à 7, **caractérisé en ce que** ledit aminoglycoside du groupe II est la streptomycine.

9. Utilisation d'un aminoglycoside du groupe II pour la précipitation, la détection et/ou le diagnostic de la PrP^{sc} même lors de la détection en immunohistochimie.

10. Utilisation *in vitro* d'un aminoglycoside du groupe II pour l'élimination de la PrP^{sc} d'un tissu ou d'un fluide biologique.

11. Utilisation d'un kit comprenant un aminoglycoside du groupe II pour le diagnostic des pathologies liées à la présence de la PrP^{sc} comme la tremblante des petits ruminants, la maladie cachectisante chronique de l'élan et de l'antilope, l'encéphalopathie spongiforme bovine et la maladie de Creutzfeldt-Jacob.

12. Utilisation selon la revendication 11, **caractérisée en ce que** ledit aminoglycoside du groupe II est la streptomycine.

## Claims

1. Method for the detection of PrP, particularly PrP^{sc}, **characterised in that** a biological tissue or fluid from or obtained from an animal or human body is placed in contact with an antibiotic selected in the group II aminoglycoside family.

2. Method according to claim 1, **characterised in that**:
a) a group II aminoglycoside is added to the suspension of a biological tissue or fluid from or obtained from an animal or human body,
b) the solution is placed in a suitable buffer, and is subjected to heating, and the solution obtained is centrifuged and the sediment is separated from the supernatant,
c) the PrP^{sc} is detected after migration on an electrophoresis gel, transfer and immunodetection.

3. Method according to claim 1, **characterised in that**:
a) proteinase K is added to the suspension of a biological tissue or fluid from or obtained from an animal or human body,
b) a group II aminoglycoside is added,
c) the suspension is placed in a suitable buffer, and is subjected to heating, and the solution obtained is centrifuged and the sediment is separated from the supernatant,
d) the PrP^{sc} is detected after migration on an electrophoresis gel, transfer and immunodetection.

4. Method according to any of claims 1 to 3, **characterised in that** said biological tissue is from or obtained from a brain or other animal or human tissue.

5. Method according to any of claims 3 or 4, **characterised in that** said proteinase is a proteinase K.

6. Method according to any of claims 1 to 5, **characterised in that** the biological tissue or fluid, prior to being placed in contact with said aminoglycoside, is homogenized in a glucose solution.

7. Method according to any of claims 1 to 6, **characterised in that** the heating step corresponds to a temperature rise between 60 and 150°C.

8. Method according to any of claims 1 to 7, **characterised in that** group II aminoglycoside is streptomycin.

9. Use of a group II aminoglycoside for the precipitation, detection and/or diagnosis of PrP^{sc} even during detection in immunohistochemistry.

10. In vitro use of a group II aminoglycoside for the elimination of PrP^{sc} from a biological tissue or fluid.

11. Use of a kit comprising a group II aminoglycoside for the diagnosis of diseases associated with the presence of PrP^{sc} such as scrapie, chronic cachexia in the moose and antelope, bovine spongiform encephalopathy and Creutzfeldt-Jakob disease.

12. Use according to claim 11, **characterised in that** said group II aminoglycoside is streptomycin.

## Patentansprüche

1. Verfahren zum Nachweis von PrP, insbesondere von PrP^{sc}, **dadurch gekennzeichnet, dass** ein biologisches Gewebe oder Fluid aus oder hergestellt aus einem tierischen oder menschlichen Organismus mit einem Antibiotikum zusammengebracht wird, das aus der Familie der Aminoglykoside der Gruppe II ausgewählt ist.

2. Verfahren nach Anspruch 1, **dadurch gekennzeichnet, dass**:
a) der Suspension eines biologischen Gewebes oder Fluids aus oder hergestellt aus einem tierischen oder menschlichen Organismus ein Aminoglykosid der Gruppe II hinzugefügt wird,
b) die Lösung in einen entsprechenden Puffer verbracht wird, und sie erhitzt wird, danach die hergestellte Lösung suspendiert wird und das Überstandskonzentrat separiert wird,
c) das PrP^{sc} nach Migration auf ein Elektrophoresegel, Transfer und Immundetektion nachgewiesen wird.

3. Verfahren nach Anspruch 1, **dadurch gekennzeichnet, dass**:
a) der Suspension eines biologischen Gewebes oder Fluids aus oder hergestellt aus einem tierischen oder menschlichen Organismus Proteinase K hinzugefügt wird,
b) ein Aminoglykosid der Gruppe II eingesetzt wird,
c) die Suspension in einen entsprechenden Puffer verbracht wird, und sie erhitzt wird, danach die hergestellte Lösung suspendiert wird und das Überstandskonzentrat separiert wird,
d) das PrP^{sc} nach Migration auf ein Elektrophoresegel, Transfer und Immundetektion nachgewiesen wird.

4. Verfahren nach einem der Ansprüche 1 bis 3, **dadurch gekennzeichnet, dass** das biologische Gewebe Hirngewebe oder ein anderes tierisches oder menschliches Gewebe ist oder daraus hergestellt.

5. Verfahren nach einem der Ansprüche 3 oder 4, **dadurch gekennzeichnet, dass** die Proteinase eine Proteinase K ist.

6. Verfahren nach einem der Ansprüche 1 bis 5, **dadurch gekennzeichnet, dass** das biologische Gewebe oder Fluid, bevor es mit dem Aminoglykosid in Kontakt gebracht wird, in einer Glukoselösung homogenisiert wird.

7. Verfahren nach einem der Ansprüche 1 bis 6, **dadurch gekennzeichnet, dass** der Schritt der Erhitzung einer Temperaturerhöhung zwischen 60 und 150 °C inklusive entspricht.

8. Verfahren nach einem der Ansprüche 1 bis 7, **dadurch gekennzeichnet, dass** das Aminoglykosid der Gruppe II Streptomycin ist.

9. Verwendung eines Aminoglykosids der Gruppe II für die Fällung, den Nachweis und/oder die Diagnose von dem PrP^{sc}, auch beim immunhistochemischen Nachweis.

10. *in vitro*-Verwendung eines Aminoglykosids der Gruppe II zur Entfernung des PrP^{sc} aus einem biologischen Gewebe oder Fluid.

11. Verwendung eines Kits, das ein Aminoglykosid der Gruppe II umfasst, zur Diagnose von Krankheiten, die mit der Anwesenheit von PrP^{sc} verbunden sind, wie die Scrapie der Kleinwiederkäuer, die chronische Auszehrung des Elchs und der Antilope, die bovine spongiforme Enzephalopathie und die Creutzfeldt-Jacob-Krankheit.

12. Verwendung nach Anspruch 11, **dadurch gekennzeichnet, dass** das Aminoglykosid der Gruppe II Streptomycin ist.
